# EUROPEAN PATENT APPLICATION

(11) **EP 2 698 144 A1**
(43) Date of publication of application: **19.02.2014**
(21) Application number: 13002861.6
(22) Date of filing: 04.06.2013
(51) Int. Cl.: A61K 9/00, A61K 9/20, A61K 47/38, A61K 31/195

(54) **Prolonged release gabapentin tablet formulation whose ability to stay in the stomach is improved**

(30) Priority: 12.08.2012 TR 201206836
(71) Applicant: Ali Raif Ilaç Sanayi ve Ticaret Anonim Sirketi, Istanbul (TR)
(72) Inventor: Bulgur, Abdullah, Istanbul (TR); Kesgin, Didehan, Istanbul (TR); Göçmen, Özlem, Istanbul (TR)
(74) Representative: Bulut, Pinar

(57) **Abstract**

It is related to long acting tablet formulation of gabapentin (dispersed in the polymer matrix) whose ability to stay in the stomach is improved thanks to its feature of swelling (when contacts with the gastric fluid) and buoyancy and which comprises hydroxypropyl methylcellulose and ethyl cellulose mixture.

## Description

### Technical Aspect:

The present invention is related to prolonged release tablet formulations whose capacity for staying in the stomach is improved thanks to the features such as dimension increase by the swelling of the tablet and the buoyancy.

### Prior Art:

Gabapentin is the structural analog of gamma amino butyric acid (GABA) which is an important neurotransmitter of central nervous system and it is a third generation antiepileptic drug. Besides partial epileptic seizures, it is also used for the treatment of neuropathic pain and movement disorders.

The molecular formula of gabapentin is C₉H₁₇NO₂ and its molecular weight is 171.237. Its chemical name is 2-[1-(aminomethyl)cyclohexyl]acetic acid (Formula I).

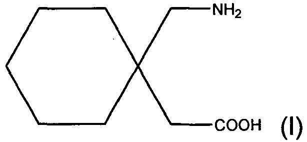

US2003/0104053 application is related to long acting gabapentin formulation which is composed of the combination of polyethylene oxide and hydroxypropyl methylcellulose, which stays in the stomach as it gets bigger by swelling when it contacts with the gastric fluid and is released in the stomach and upper stomach-intestine region.

In application no US2009/0017121, a dosage form, which comprises at least one swellable hydrophilic polymer and thus dimensionally grows by swelling in water and provides the staying of the dosage form in stomach when the patient is full, comprises 100 mg to 4800 mg gabapentin dispersed in polymer matrix and in which one hour after the administration, at least 40% of gabapentin is still present in the dosage form and which releases gabapentin during at least 5 hours from the dosage form by diffusion, is described.

In application no. US2012/0064129, a tablet formulation, which comprises at least one hydrophilic membrane, swells when contacts with water and has a bioavailability of at least 80% compared to instant release tablets, is described.

### Description of the Invention:

The present invention is related to long acting tablet formulation of gabapentin (dispersed in the polymer matrix) whose ability to stay in the stomach is improved thanks to its swelling (when contacts with the gastric fluid) and buoyancy features.

For long acting tablets, increasing the staying time in the stomach is important in terms of efficiency as the gabapentin is absorbed only in the exit of stomach.

The exit time of the tablet from the stomach is related to the absorption of the active substance. As the control of the exit of the products of the patents mentioned above from the stomach by swelling causes alterations in the blood concentrations among the individuals, it is targeted to control the exit from the stomach by more than one method and consequently, in addition to swelling, buoyancy of the tablet is provided by the addition of some excipients. Sodium bicarbonate used for this purpose reacts with the gastric acid and as a result of the carbon dioxide (gas) release, spaces formed within the tablet provide the buoyancy of the tablet in the gastric fluid and thus, exiting of the tablet from the stomach is delayed. Other inorganic carbonate and/or bicarbonate compounds may be used instead of sodium bicarbonate.

Accordingly, the prolonged release gabapentin formulation comprises hydroxypropyl methylcellulose and ethyl cellulose mixture as the polymeric matrix controlling the active substance release.

Thus, the prolonged release gabapentin formulation core tablet comprises hydroxypropyl methylcellulose and ethyl cellulose as the agent controlling the release, polyethylene glycol as the hydrophilic agent, sodium bicarbonate as the gas forming agent and magnesium stearate as the lubricant. The prepared core tablet is coated with a suitable coating agent.

In the core tablet, Methocel K 15M, whose viscosity in 2% aqueous solution is 11.250-21.000 cp, is used as hydroxypropyl methylcellulose.

Core tablet is produced by wet granulation technique. Examples below are for the explanation of the invention, in no way they restrict it.

### EXAMPLES :

According to the present invention, the formulations below are studied. All the formulations are manufactured by wet granulation.

In order to control the similarity of Gabapentin with the original product during the tests, "Gralise Tablet" is used as the reference product. The similarity factor (f2), which is used for comparing dissolution profiles of the test formulation and the reference product, is a measure for the similarity of the two dissolution graphics and when the f2 value is equal to or bigger then 50, the dissolution profile is accepted as similar.

**Table 1. Test formulations (quantities are given in mg)**

| Ingredients | I | II |
|---|---|---|
| Gabapentin | 600.0 | 300.0 |
| Ethyl cellulose | 100.0 | 250.0 |
| PEG 6000 | 40.0 | 20.00 |
| Methocel K15 M | 215.0 | 107.50 |
| Sodium bicarbonate | 40.0 | 20.00 |
| Magnesium stearate | 5.0 | 2.50 |
| Total | 1000 | 700 |

The prepared tablet is coated with a suitable coating agent in such a way that its weight increases 2%.

Formulations are tested at 37°C, in 900ml dissolution medium, 100rpm speed, basket method dissolution conditions. pH 1.2 stomach medium, deionized water and pH 4.5 acetate buffer with pH 6.8 phosphate buffer are used as the disolution medium. It was found out that, the f2 values of the formulations are higher than 50 and therefore, they are similar with the original products.

The results of the dissolution tests are given in the tables below.

**Table 2. Dissolution test results of reference product-Formula 1 pH 1.2 gastric medium (%)**

| | 1 hours | 2 hours | 4 hours | 8 hours | 10 hours | 12 hours | F2 |
|---|---|---|---|---|---|---|---|
| Formula 1 | 21.87 | 33.84 | 50.58 | 73.15 | 81.26 | 87.33 | 77.6 |
| Reference Product | 19.78 | 31.30 | 49.08 | 75.96 | 84.53 | 90.40 | |

**Table 3. Dissolution test results of reference product-Formula 2 pH 1.2 gastric medium (%)**

| | 1 hour | 2 hours | 4 hours | 8 hours | 10 hours | 12 hours | F2 |
|---|---|---|---|---|---|---|---|
| Formula 2 | 25.71 | 40.49 | 60.16 | 83.43 | 91.03 | 95.66 | 70.0 |
| Reference Product | 21.95 | 34.40 | 54.95 | 80.87 | 89.25 | 94.58 | |

**Table 4. Dissolution test results of pH 6.8 phosphate buffer (%)**

| | 1 hour | 2 hours | 4 hours | 8 hours | 10 hours | 12 hours | F2 |
|---|---|---|---|---|---|---|---|
| Formula 1 | 19.58 | 31.19 | 46.77 | 68.50 | 76.69 | 83.38 | 63.6 |
| Reference Product | 16.31 | 25.93 | 40.71 | 62.62 | 71.25 | 78.22 | |

**Table 5. Dissolution test results of deionized water (%)**

| | 1 hour | 2 hours | 4 hours | 8 hours | 10 hours | 12 hours | F2 |
|---|---|---|---|---|---|---|---|
| Formula 1 | 21.24 | 33.80 | 52.22 | 74.67 | 82.80 | 88.96 | 51.8 |
| Reference Product | 16.51 | 26.25 | 41.25 | 64.00 | 72.62 | 79.68 | |

**Table 6. Dissolution test results of pH 4.5 acetate buffer (%)**

| | 1 hour | 2 hours | 4 hours | 8 hours | 10 hours | 12 hours | F2 |
|---|---|---|---|---|---|---|---|
| Formula 1 | 18.49 | 27.75 | 41.20 | 62.33 | 70.74 | 77.25 | 86.0 |
| Reference Product | 16.14 | 25.98 | 40.67 | 62.86 | 72.23 | 79.36 | |

### List of Figures :

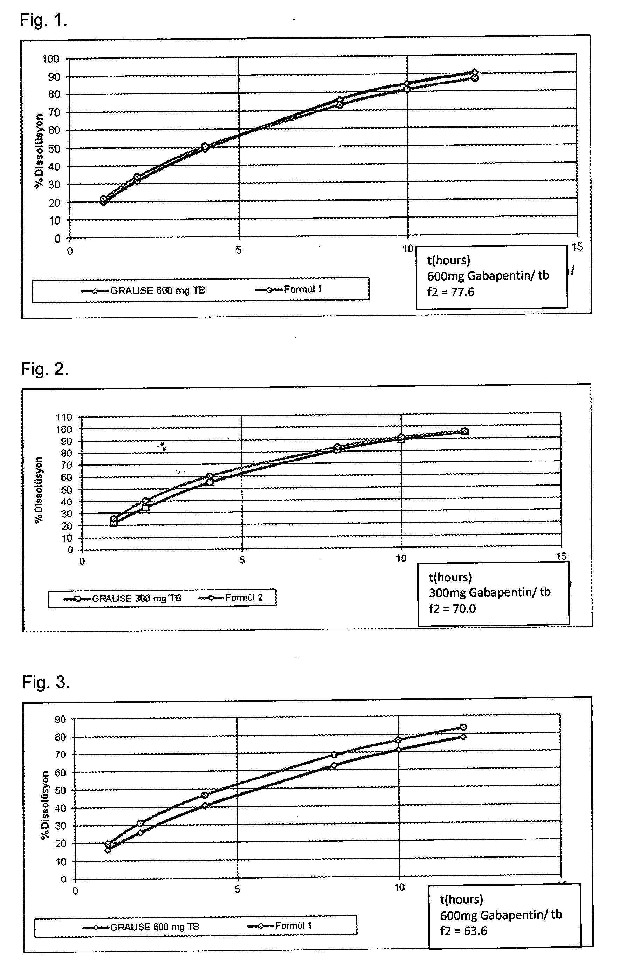

Figure 1. Dissolution profiles of reference product and Formula 1 (pH 1.2 gastric medium)
Figure 2. Dissolution profiles of reference product and Formula 2 (pH 1.2 gastric medium)
Figure 3. Dissolution profiles of reference product and Formula 1 (pH 6.8 phosphate buffer)

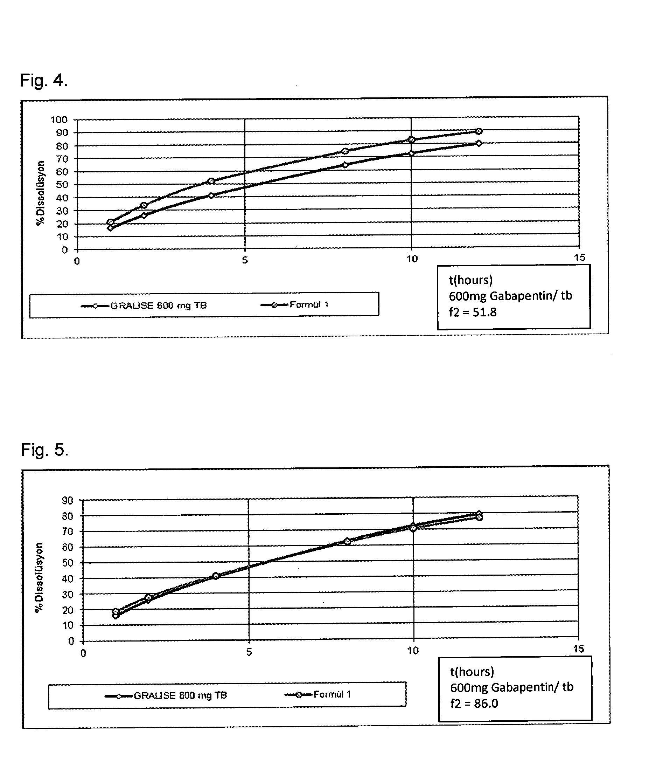

Figure 4. Dissolution profiles of reference product and Formula 1 (Deionized water)
Figure 5. Dissolution profiles of reference product and Formula 1 (pH 4.5 acetate buffer)

## Claims

1. Prolonged release gabapentin formulation dispersed in polymer matrix **characterized in that** it comprises at least one gas releasing agent with hydroxypropyl methylcellulose and ethyl cellulose mixture as polymer matrix in order to improve the ability to stay in the stomach thanks to its ability to swell and float when it contacts with the gastric fluid.

2. Core tablet of prolonged release gabapentin formulation according to Claim 1 **characterized in that** it comprises inorganic carbonate and/or bicarbonate compounds as gas releasing agent.

3. Core tablet of prolonged release gabapentin formulation according to Claim 1 **characterized in that** it comprises sodium bicarbonate as gas releasing agent.

4. Core tablet of prolonged release gabapentin formulation according to Claim 1 **characterized in that** it comprises hydroxypropyl methylcellulose and ethyl cellulose as the agent controlling the release, polyethylene glycol as the hydrophilic agent, sodium bicarbonate as the gas forming agent and magnesium stearate as the lubricant.

5. Core tablet of prolonged release gabapentin formulation according to Claim 1 **characterized in that** it is prepared with wet granulation technique.

6. Core tablet of prolonged release gabapentin formulation according to Claim 1 **characterized in that** it comprises hydroxypropyl methylcellulose, whose viscosity in 2% aqueous solution is 11.250-21.000 cp.

7. Core tablet of prolonged release gabapentin formulation according to Claim 1 **characterized in that** it is coated with a convenient coating agent.
